(19) Europäisches Patentamt
European Patent Office
Office européen des brevets

(11) EP 1 607 476 B1

(12) # EUROPEAN PATENT SPECIFICATION

(45) Date of publication and mention of the grant of the patent:
**28.11.2007 Bulletin 2007/48**

(51) Int Cl.:
***C12N 1/00*** (2006.01)

(21) Application number: **05012049.2**

(22) Date of filing: **03.06.2005**

(54) **A microorganism separated from Kefir grains, a microorganism culture obtained by culturing said microorganism or microorganisms including it, and a product using such microorganisms or microorganism cultures**

Mikroorganismus isoliert vom Kefir, die Mikroorganismuskultur und die Produkte die diesen Mikroorganismus oder die Mikroorganismuskultur enthalten

Microorganisme isolé de Kéfir, la culture de microorganismes du même et les produits qui contiennent ce microorganisme ou cette culture de microorganismes

(84) Designated Contracting States:
**AT BE BG CH CY CZ DE DK EE ES FI FR GB GR HU IE IS IT LI LT LU MC NL PL PT RO SE SI SK TR**

(30) Priority: **14.06.2004 JP 2004175969**

(43) Date of publication of application:
**21.12.2005 Bulletin 2005/51**

(73) Proprietors:
- **K.K. Yonezawa Biru Shisutemu Sabisu Kanazawa-shi Ishikawa-ken (JP)**
- **Takeda, Kazunori Hakusan-shi Ishikawa-ken (JP)**

(72) Inventor: **Takeda, Kazunori Ishikawa-ken (JP)**

(74) Representative: **Glawe, Delfs, Moll Patentanwälte Postfach 26 01 62 80058 München (DE)**

(56) References cited:
**WO-A-01/97820** **FR-A- 2 580 150**

- **PATENT ABSTRACTS OF JAPAN vol. 2003, no. 12, 5 December 2003 (2003-12-05) & JP 2003 310154 A (NIPPON KEFIA KK), 5 November 2003 (2003-11-05)**
- **DATABASE EMBL [Online] 8 February 1992 (1992-02-08), "Lactobacillus mali 16S ribosomal RNA." XP002345723 retrieved from EBI accession no. EM_PRO:LMRR16SV Database accession no. LMRR16SV**
- **DATABASE EMBL [Online] 23 January 2005 (2005-01-23), "Lactobacillus sp. 71 16S ribosomal RNA gene, complete sequence." XP002345724 retrieved from EBI accession no. EM_PRO: AY681129 Database accession no. AY681129**
- **DATABASE EMBL [Online] 16 July 2003 (2003-07-16), "Lactobacillus sp. CECT 5924 16S rRNA gene, strain CECT 5924" XP002345725 retrieved from EBI accession no. EM_PRO: LSP576009 Database accession no. LSP576009**
- **DATABASE EMBL [Online] 18 September 2004 (2004-09-18), "Lactobacillus satsumensis gene for 16S rRNA, partial sequence, strain:NRIC 0604." XP002345726 retrieved from EBI accession no. EM_PRO:AB154519 Database accession no. AB154519**
- **DATABASE EMBL [Online] 11 February 2004 (2004-02-11), "Lactobacillus nagelii gene for 16S rRNA, partial sequence." XP002345727 retrieved from EBI accession no. EM_PRO:AB162131 Database accession no. AB162131**
- **SANTOS A ET AL: "The Antimicrobial Properties of Different Strains of Lactobacillus spp. Isolated from Kefir" SYSTEMATIC AND APPLIED MICROBIOLOGY, XX, XX, vol. 26, no. 3, 2003, pages 434-437, XP004957460 ISSN: 0723-2020**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **PATENT ABSTRACTS OF JAPAN vol. 2003, no. 12, 5 December 2003 (2003-12-05) & JP 2004 149442 A (YONEZAWA BIRU SYSTEM SERVICE: KK), 27 May 2004 (2004-05-27)**
- **M. AHOTUPA, M.SAXELIN, R. KORPELA: "Antioxidative properties of lactobacillus GG" NUTRITION TODAY SUPPLEMENT, vol. 31, no. 6, November 1996 (1996-11), pages 51S-52S, XP009053199**

Sequence Listing

<110> Kabushiki Kaisha Yonezawa Biru Shisutemu Sabisu
<120> A microorganism separated from Kefir grains, a microorganism culture obtained by culturing this microorganism or microorganisms including it, and a product using them.

<130> WM/et/es-15738

<150> JP 2004-175 369
<151> 2004-06-14

<160> 1

<210> 1
<211> 1521
<212> DNA
<213> Lactobacillus mali

<400> 1

```
gagtttgatc  ctggctcagg  acgaacgctg  gcggcgtgcc  taatacatgc    50
aagtcgtacg  cagtttcttc  accgagtgct  tgcactcacc  gaagaaactg   100
agtggcgaac  gggtgagtaa  cacgtgggta  acctgcccaa  aagaggggga   150
taacacttgg  aaacaggtgc  taataccgca  taacaacaaa  aaccgcctgg   200
tttttgttta  aaagatggtt  tcggctatca  cttttggatg  gacccgcggc   250
gtattagcta  gttggtaagg  taatggctta  ccaaggcagt  gatacgtagc   300
cgaactgaga  ggttgatcgg  ccacattggg  actgagacac  ggcccaaact   350
cctacgggag  gcagcagtag  ggaatcttcc  acaatggacg  caagtctgat   400
ggagcaacgc  cgcgtgagtg  aagaaggttt  tcggatcgta  aaactctgtt   450
gttagagaag  aacgtgtgtg  agagtaactg  ttcatrcagt  gacggtatct   500
aaccagaaag  ccacggctaa  ctacgtgcca  gcagccgcgg  taatacgtag   550
gtggcaagcg  ttgtccggat  ttattgggcg  taaagggaac  gcaggcggtc   600
ttttaagtct  gatgtgaaag  ccttcggctt  aaccgaagtc  gtgcattgga   650
aactgggaga  cttgagtgca  gaagaggaga  gtggaactcc  atgtgtagcg   700
gtgaaatgcg  tagatatatg  gaagaacacc  agtggcgaaa  gcggctctct   750
ggtctgtaac  tgacgctgag  gttcgaaagc  gtgggtagca  aacaggatta   800
gataccctgg  tagtccacgc  tgtaaacgat  gaatgctaag  tgttggaggg   850
tttccgccct  tcagtgctgc  agctaacgca  ttaagcattc  cgcctgggga   900
gtacgaccgc  aaggttgaaa  ctcaaaggaa  ttgacggggg  cccgcacaag   950
cggtggagca  tgtggtttaa  ttcgaagcaa  cgcgaagaac  cttaccaggt  1000
cttgacatct  tttgctaacc  tgagagatca  ggygttccct  tcggggacaa  1050
aatgacaggt  ggtgcatggt  tgtcgtcagc  tcgtgtcgtg  agatgttggg  1100
ttaagtcccg  caacgagcgc  aacccttatt  actagttgcc  agcatttagt  1150
tgggcactct  agtgagactg  ccggtgacaa  accggaggaa  ggtggggatg  1200
acgtcaaatc  atcatgcccc  ttatgacctg  ggctacacac  gtgctacaat  1250
ggacggtaca  acgagtcgca  agaccgcgag  gtttagctaa  tctcttaaaa  1300
ccgttctcag  ttcggattgt  aggctgcaac  tcgcctacat  gaagtcggaa  1350
tcgctagtaa  tcgcggatca  gcatgccgcg  gtgaatacgt  tcccgggcct  1400
tgtacacacc  gcccgtcaca  ccatgagagt  ttgtaacacc  caaagccggt  1450
gcggtaacct  tttaggagcc  agccgtctaa  ggtgggacag  atgattgggg  1500
tgaagtcgta  acaaggtagc  c                                  1521
```

## Description

[TECHNICAL FIELD OF INVENTION]

The present invention relates to an antioxidant microorganism derived from a natural substance, used as a raw material of, or added to, drinks, foods, cosmetics and animal feeds, thereby preventing their deterioration due to oxidation, and scavenging or inhibiting generation of active oxygens and free radicals that are generated in vivo of humans and animals that use such drinks, foods, cosmetics or animal feeds. The present invention also relates to a microorganism culture obtained by culturing said microorganism or microorganisms including it. The present invention further relates to drinks, foods, cosmetics and animal feeds using such microorganisms or microorganism cultures.

[PRIOR ART]

Lifestyle-related diseases such as malignant neoplasm (cancer), cerebrovascular diseases and diabetes are among the top causes of death in recent years. Major causes of lifestyle-related diseases include active oxygens and free radicals. There are different kinds of antioxidants: those added to drinks, foods, cosmetics and animal feeds to prevent their deterioration and preserve their quality; and those used to scavenge or inhibit generation of active oxygens and free radicals that are generated in vivo.

When oils and fats contained in a drink, food, cosmetic or animal feed product are oxidized, they not only affect the product’s color and flavor, but they also cause the product’s color to brown or fade and its nutrition value to decline. For this reason, many drinks, foods, cosmetics and animal feeds are added with antioxidants to preserve their quality.

Antioxidants used for such purposes in the fields of drinks, foods and animal feeds include natural antioxidants such as L-ascorbic acid (vitamin C), erythorbate (isoascorbic acid) and tocopherol (vitamin E) as well as phenolic synthetic antioxidants such as Di-n-butyl hydroxytoluene (BHT) and butylated hydroxyanisole (BHA).

[DISCLOSURE OF THE INVENTION]

[OBJECTIVES TO BE ACHIEVED BY THE INVENTION]

L-ascorbic acid easily dissolves in water and is often used for its strong oxido-reduction properties. It is, however, difficult to be used in edible oil and fat because its acid (acidity) changes the taste of the food. For this reason, the use of L-ascorbic acid is limited to processed fruits and pickles, etc. Tocopherols are often used because of their ability to prevent unnecessary oxidation of oil ingredients. Their antioxidant properties, however, are not very strong, so they need to be used in combination with other antioxidants.

Natural antioxidants are generally inferior to synthetic antioxidants with respect to their antioxidant properties. On the other hand, while synthetic antioxidants such as Di-n-butyl hydroxytoluene (BHT) and butylated hydroxyanisole (BHA) have strong antioxidant properties, they are considered dubious food additives because of their cancer-inducing properties as exemplified by gene injury, mutagenicity and abnormal chromosomes. Their safety has been questioned.

Both natural and synthetic antioxidants are used in cosmetics to prevent oxidation of oils and fats used in them. But these antioxidants are considered to have adverse effects on the skin; they cause rough skin. Their safety has also been questioned.

For the above reasons, development of an antioxidant that has strong antioxidant properties and is highly versatile and derived from a natural substance has been desired.

Natural substances that have been reported to exhibit antioxidant properties include browning substances, amino acids, sugar alcohols, fructose (fruit sugar), fruit peels, wasabi (Japanese horseradish) and catechins. Antioxidants derived from microorganism cultures include lactic acid bacteria extracts that exhibit antioxidant effects.

In the past, these antioxidants were added to drinks, foods, cosmetics and animal feeds for the purposes of preventing their deterioration and preserving their quality. These antioxidants have also been found to scavenge or inhibit generation of active oxygens and free radicals that are generated in vivo. To take advantage of these properties, natural extracts with strong in vivo antioxidant properties have been sought.

Four kinds of active oxygens are known that are generated by in vivo oxidation. They are: singlet oxygens, super oxides, hydroxyl radicals and hydrogen peroxides. More than ten kinds of free radicals including alkyl hydro peroxides and hydro-peroxyl radicals are known that are generated by in vivo oxidation. The antioxidant properties can be determined by measuring the radical-scavenging capacity using 1, 1-diphenyl-2-picrylhydrazine (DPPH).

In vivo oxidation is considered to be a cause that triggers aging, lifestyle-related diseases and cancers. Aging causes weakening of physical functions not so much as sickness, such as hypokinesis, hypofunction of internal organs, failing memory, cataract and wrinkles. It also causes blood vessels to deteriorate, increasing the vulnerability to arterial sclerosis. Lifestyle-related diseases that are considered especially relevant to in vivo oxidation include arterial sclerosis and diabetes, as well as liver damage and rheumatic arthritis.

As for the relevance to cancer, in vivo oxidation is considered to be one of the causes that transmute and damage the substances that constitute DNA and mutate other cells when they are regenerated. In order to prevent such diseases, antioxidants that prevent or inhibit in vivo oxidation are drawing attention. They are researched and developed in the forms of foods with function claims, foods for specified health uses and supplements.

Antioxidants for such purposes include vegetable derivatives such as carotenoids (beta carotene, lycopene, fucoxanthine), phenolic compounds (flavonoid, anthocyanin, catechin), sulphides and beta diketones, and microorganism cultures such as DHA (docosahexaenoic acid) and EPA (eicosapentaenoic acid).

One objective of the present invention is to provide a microorganism that is a natural extract with strong antioxidant properties, highly versatile for drinks, foods, cosmetics and animal feeds, and capable of maintaining the product's original taste and flavor while having in vivo antioxidant properties, as well as providing a microorganism culture that is obtained by culturing this microorganism. Another objective of the present invention is to provide various drinks, foods, cosmetics and animal feeds that use such microorganisms.

Kefir is a traditional compound fermented milk product of lactic acid bacteria and yeast organisms thought to have originated in Caucasus, and is made by culturing Kefir grains in milk.

The inventor of the present invention discovered that natural Kefir grains make safe products for humans and animals when used in drinks, foods, cosmetics and animal feeds, and continued research into various uses of Kefir. The inventor further discovered that a cultured liquid made by culturing Kefir grains in a green-tea extract and removing bacteria from the microorganism culture had a skin-whitening effect and that Kefir grains themselves had antioxidant properties. Based on these findings, the inventor applied for a patent in the form of a cosmetic that takes advantage of such properties of Kefir. (Japanese Patent Application No. 2002-315520, Application filing date: October 30, 2002) and Publication Number JP 2004149442 (2004.05.27).

As a result of further research, the inventor has identified a microorganism derived from a natural substance and having antioxidant properties that can be used as a raw material of, or added to, drinks, foods, cosmetics and animal feeds, thereby preventing their deterioration due to oxidation and scavenging or inhibiting generation of active oxygens and free radicals that are generated in vivo of humans and animals. The inventor has also discovered that a microorganism culture obtained by culturing said microorganism or microorganisms is capable of achieving the above objectives. The inventor has also come to develop a product having antioxidant properties by adding the microorganisms in drinks, foods, cosmetics and animal feeds.

[MEANS FOR ACHIEVING THE OBJECTIVES]

The present invention relates to:

(1) a new species of microorganism having antioxidant properties that is separated from Kefir grains and belongs to Lactobacillaceae SIID 1719-6b;
(2) a microorganism culture having antioxidant properties obtained in a single culture of a new species of microorganism that is separated from Kefir grains and belongs to Lactobacillaceae;
(3) a microorganism culture having antioxidant properties that is obtained by culturing microorganisms separated from Kefir grains and belongs to Lactobacillaceae SIID 1719-6b;
(4) a drink having antioxidant effects added with cultures or bacteria obtained by culturing microorganisms separated from Kefir grains and a new species of microorganism belonging to Lactobacillaceae SIID 1719-6b;
(5) a food having antioxidant effects added with cultures or bacteria obtained by culturing microorganisms separated from Kefir grains and a new species of microorganism belonging to Lactobacillaceae SIID 1719-6b;
(6) a cosmetic having antioxidant effects added with cultures or bacteria obtained by culturing microorganisms separated from Kefir grains and a new species of microorganism belonging to Lactobacillaceae SIID 1719-6b; and
(7) an animal feed having antioxidant effects added with cultures or bacteria obtained by culturing microorganisms separated from Kefir grains and a new species of microorganism belonging to Lactobacillaceae SIID 1719-6b.

The "antioxidant properties" is defined as including the ability to prevent oxidization thereby preserving the quality of the product as well as the ability to scavenge or inhibit active oxygens and free radicals that are generated in vivo thereby preventing in vivo oxidization when given to humans or animals as drinks, foods or animal feeds. The "microorganism culture" means a culture of microorganisms or a single microorganism separated from Kefir grains.

[EFFECTS OF THE INVENTION]

The invention as described in claim 1 has the effect of providing a new species of microorganism having antioxidant properties that is separated from Kefir grains and belongs to Lactobacillaceae SIID 1719-6b. By using this microorganism, which has antioxidant properties, as a raw material for drinks, foods, cosmetics or animal feeds, or by adding it to these products or their raw materials, it is possible to prevent their deterioration due to oxidation and to scavenge or inhibit generation of active oxygens and free radicals that are generated in vivo of humans and animals that use them.

The invention as described in claim 2 has the effect of providing a microorganism culture having antioxidant properties that includes a new species of microorganism separated from Kefir grains and belonging to Lactobacillaceae SIID 1719-6b. This culture, which is not toxic and has no adverse effect, has a variety of applications including raw materials and additives for drinks, foods, cosmetics, animal feeds, etc.

The invention as described in claim 3 has the effect of providing a microorganism culture having antioxidant properties that is obtained by culturing microorganisms separated from Kefir grains belonging to Lactobacillaceae SDIID 1719-6b. This culture, which is not toxic and has no adverse effect, has a variety of applications including raw materials and additives for drinks, foods, cosmetics and animal feeds.

The inventions as described in claims 4 to 7 has the effects of providing antioxidant drinks, foods, cosmetics and animal feeds that are added with cultures or bacteria obtained by culturing microorganisms separated from Kefir grains and a new species of microorganism belonging to Lactobacillaceae SIID 1719-6b.

[BRIEF DESCRIPTION OF THE DRAWINGS]

FIG. 1 is a graph showing the relationship between the culture time and the growth of the microorganism.
FIG. 2 is a graph showing the relationship between the culture time (growth of the microorganism) and the changes of pH.
FIG. 3 is a graph showing the relationship between the culture time (growth of the microorganism) and the antioxidant properties.
FIG. 4 is a photograph of Gram stained SIID1719-6b.
FIG. 5 shows the results of the molecular pedigree analysis of SIID1719-6b.

[BEST EMBODIMENTS OF THE INVENTION]

Preferred embodiments of the present invention will now be explained.

Strains of microorganisms separated from Kefir grains include Acetobacter cerevisiae SIID1719-2b, Gluconobacter oxydans SIID1719-3b, Lactobacillus sp. SIID1719-6b, which is a new species of the Lactobacillaceae family, Pichiamembrani faciens SIID1719-1y, Saccharomyces cerevisiae SIID1719-4y and Picchia anomala SIID1719-5y.

In order to find out whether microorganisms separated from Kefir grains and a microorganism culture obtained in a single culture of Lactobaccilus sp. SIID1719-6b, which is a new species of the strain Lactobacillaceae family, have antioxidant properties, we conducted the following evaluation tests. As a result, we found that these microorganisms and the Lactobacillus sp. SIID1719-6b itself have antioxidant properties. Moreover, active principles of the culture that is removed of bacteria in a centrifugal separator and sterilized using a sterilizing filter were also found to have antioxidant properties. According to the present invention, anything that shows the same properties in symbiosis with microorganisms whose safety with Lactobacillus sp. SIID1719-6b is confirmed can be used. The Lactobacillus sp. SIID1719-6b, which is a new species of the Lactobacillaceae family, is deposited at the International Patent Organism Depository of the National Institute of Advanced Industrial Science and Technology.

As a typical example, a strain of Lactobacillus sp. SIID1719-6b, which is a new species of the family Lactobacillaceae, was cultured and its antioxidant properties were evaluated as described in the following paragraphs.

A strain of Lactobacillus sp. SIID1719-6b, which is a new species of the family Lactobcillaceae, is cultured in a medium M.R.S. Broth (Oxoid England, UK). The composition of the medium M.R.S. is shown in table 1. $2 \times 10^6$ cells/ml of bacteria are cultured statically at a culture temperature of 30˚C and sampled every 24 hours to measure the number of bacteria and pH. The growth changes in the number of bacteria are shown in FIG. 1, and the changes of pH are shown in FIG. 2. There is no limitation as to the medium composition, substrate and temperature of the culture as long as the conditions allow the bacteria to grow well.

Table 1
Composition of the MRS medium in 1 liter of purified water

| Ingredients of the medium | Amount added |
|---|---|
| Peptone | 10.0 g |

(continued)

Composition of the MRS medium in 1 liter of purified water

| Ingredients of the medium | Amount added |
|---|---|
| Lab-Lemco' powder | 8.0 g |
| Yeast extract | 4.0 g |
| Glucose | 20.0 g |
| Sorbitan mono-oleate | 1 ml |
| Dipotassium hydrogen phosphate | 2.0 g |
| Sodium acetate $3H_2O$ | 5.0 g |
| Triammonium citrate | 2.0 g |
| Magnesium sulphate $7H_2O$ | 0.2 g |
| Manganese sulphate $4H_2O$ | 0.05 g |

We tested the antioxidant properties of the above culture. First, 190μl of 0.1 mM DPPH (dissolved in 100% ethanol) is added to each of 96 wells on a microplate. Next, samples of the culture (0, 24, 48, 72, 96 and 120 hours) and 10μl of the MRS medium (blank) are added to each well to start radical-scavenging reactions. The light absorption immediately after (0 minute) and 30 minutes after the addition of the samples were measured with a microplate reader. The radical-scavenging rate was calculated from the light absorption of the blanks and samples.

The results are shown in FIG. 3. As the culture time becomes longer, the microorganisms grow and their pH changes. As the microorganisms grow, the antioxidant properties also become more evident.

We have identified Lactobaccilus sp. SIID1719-6b to be of a new species. Our method of identification and the results of the microorganisms test are described in the following paragraphs.

As a first-stage bacteria test, we observed the cell shape, Gram stainability, the existence of spores and the motility of flagella using an optical microscope (U-LH1000, Olympus, Japan). We also observed the shapes of colonies on an MRS Broth (Oxoid, England, UK) + agar. We tested for catalase reactions, oxidase reactions, acid and gas generation from glucose and oxidation and fermentation of glucose.

The results of the mycological properties test are shown in table 2, and a photo of a Gram stained culture is shown in FIG. 4.

Table 2

Results of the mycological properties test

| Test item | | Result | |
|---|---|---|---|
| Cell shape | | Bacillus (06-0.7 x 1.5-2.0μm) | |
| Gram stain | | | + |
| Spore | | | - |
| Motility | | | - |
| Colony shape | | Culture time: 24 h<br>Circular<br>Smooth edges<br>Convex<br>Glossy<br>Milk white | |
| Culture temperature ˚C | 37 | | + |
| | 45 | | - |
| Catalase | | | - |
| Oxidase | | | - |
| Acid/gas generation (glucose) | | | +/- |
| Oxidation-fermentation (O/F) test (glucose) | | | +/+ |

We conducted a physiological and biochemical test. The results are shown in table 3.

Table 3

Physiological and biological test results

| Ingredients | Result | Ingredients | Result | Ingredients | Result |
|---|---|---|---|---|---|
| Glycerol* | - | Mannitol* | + | Raffinose* | - |
| Erythritol* | - | Sorbitol* | + | Starch* | - |
| D-arabinose* | - | α-methyl-D-mannose* | - | Glycogen* | - |
| L-arabinose* | - | α-methyl-D-glucose* | + | Xylitol* | - |
| Ribose* | - | N-acetylglucosamine* | + | Gentiobiose* | + |
| D-xylose* | - | Amygdalin* | - | D-Turanose* | + |
| L-xylose* | - | Arbutin* | + | D-Lyxose* | - |
| Adonitol* | - | Esculin* | + | D-tagatose* | - |
| β-methyl-D-xylose* | - | Salicin* | + | D-fucose* | - |
| Galactose* | - | Cellobiose* | - | L-fucose* | - |
| Glucose* | + | Maltose* | - | D-arabitol* | - |
| Fructose* | + | Lactose* | - | L-arabitol* | - |
| Mannose* | + | Melibiose* | - | Gluconate* | - |
| Sorbose* | + | Saccharose* | + | 2-ketogluconic acid* | - |
| Rhamnose* | + | Trehalose* | + | 5-ketogluconic acid* | - |
| Dulcitol* | - | Inulin* | - | | |
| Inositol* | - | Melezitose* | - | | |

We used the PrepMan Method (Applied Biosystems, CA, USA) for extraction of genome DNAs. Using the extracted genome DNAs as templates, we amplified the region of approximately 1500 to 1600 bp of all of the base sequence of 16S Ribosomal RNA genes (16S rDNA) by PCR. After that, we sequenced the amplified 16S rDNAs and obtained a base sequence. For purification of PCR products and cycle sequence, we used the MicroSeq Full 16S rDNA Bacterial Sequencing Kit (Applied Biosystems, CA, USA) (MicroSeq is a registered trademark). For the thermal cycler, we used the GeneAmp PCR SYSTEM 9600 (Applied Biosystems, CA, USA), and for the DNA sequencer, we used the ABI PRISM 3100 DNA Sequencer (Applied Biosystems, CA, USA). For the basic operations from the extraction of genome DNAs to cycle sequence, we followed the AppliedBiosystems' protocol (P/N4308132 Rev. A).

The base sequence of 16S rDNA of SIID1719-6b is shown in table 4.

Table 4

(16S rDNA base sequence of SIID1719-6b)

Sequence length: 1521

Sequence type: DNA

Name of organism: Lactobacillus mali

## Sequence

```
gagtttgatc ctggctcagg acgaacgctg gcggcgtgcc taatacatgc        50
aagtcgtacg cagtttcttc accgagtgct tgcactcacc gaagaaactg       100
agtggcgaac gggtgagtaa cacgtgggta acctgcccaa aagaggggga       150
taacacttgg aaacaggtgc taataccgca taacaacaaa aaccgcctgg       200
tttttgttta aaagatggtt tcggctatca cttttggatg gacccgcggc       250
gtattagcta gttggtaagg taatggctta ccaaggcagt gatacgtagc       300
cgaactgaga ggttgatcgg ccacattggg actgagacac ggcccaaact       350
cctacgggag gcagcagtag ggaatcttcc acaatggacg caagtctgat       400
ggagcaacgc cgcgtgagtg aagaaggttt tcggatcgta aaactctgtt       450
gttagagaag aacgtgtgtg agagtaactg ttcatrcagt gacggtatct       500
aaccagaaag ccacggctaa ctacgtgcca gcagccgcgg taatacgtag       550
gtggcaagcg ttgtccggat ttattgggcg taaagggaac gcaggcggtc       600
ttttaagtct gatgtgaaag ccttcggctt aaccgaagtc gtgcattgga       650
aactgggaga cttgagtgca gaagaggaga gtggaactcc atgtgtagcg       700
gtgaaatgcg tagatatatg gaagaacacc agtggcgaaa gcggctctct       750
ggtctgtaac tgacgctgag gttcgaaagc gtgggtagca aacaggatta       800
gataccctgg tagtccacgc tgtaaacgat gaatgctaag tgttggaggg       850
tttccgccct tcagtgctgc agctaacgca ttaagcattc cgcctgggga       900
gtacgaccgc aaggttgaaa ctcaaaggaa ttgacggggg cccgcacaag       950
cggtggagca tgtggtttaa ttcgaagcaa cgcgaagaac cttaccaggt      1000
cttgacatct tttgctaacc tgagagatca ggygttccct tcggggacaa      1050
aatgacaggt ggtgcatggt tgtcgtcagc tcgtgtcgtg agatgttggg      1100
ttaagtcccg caacgagcgc aacccttatt actagttgcc agcatttagt      1150
tgggcactct agtgagactg ccggtgacaa accggaggaa ggtggggatg      1200
acgtcaaatc atcatgcccc ttatgacctg ggctacacac gtgctacaat      1250
ggacggtaca acgagtcgca agaccgcgag gtttagctaa tctcttaaaa      1300
ccgttctcag ttcggattgt aggctgcaac tcgcctacat gaagtcggaa      1350
tcgctagtaa tcgcggatca gcatgccgcg gtgaatacgt tcccgggcct      1400
tgtacacacc gcccgtcaca ccatgagagt ttgtaacacc caaagccggt      1450
gcggtaacct tttaggagcc agccgtctaa ggtgggacag atgattgggg      1500
tgaagtcgta acaaggtagc c                                     1521
```

Using BLAST, we conducted a homology search of the above base sequence by comparing it against the DNA base sequence database, GenBank. From the base sequence of the 16S rDNA that has been obtained, we conducted a homology search for species that are considered to be close relatives of the samples and identified the top five strains. The results of the homology search are shown in table 5.

Table 5

| Homology search results | | As of May 10, 2004 | |
|---|---|---|---|
| Entries | Strains | Accession No. | Identity |
| Lactobacillus mali | | M58824 | 1424/1495 = 95.3% |
| Lactobacillus sp. CECT5924 | CECT5924 | AJ576009 | 1364/1407 = 96.9% |
| Lactobacillus nageli | NRIC0559 | AB162131 | 1440/1511 = 95.3% |
| Lactobacillus sp. | | AB016864 | 1317/1364 = 96.6% |
| Pediococcus damnosus | DSM20331 | AJ318414 | 1351/1423 = 94.9% |

Based on the information obtained so far, we constructed a molecular pedigree using the 16S rDNA base sequence and the 16S rDNA base sequence of strains that are considered to be their close relatives. We downloaded the base sequence alignment and the 16S rDNAs used in the construction of the molecular pedigree from the GenBank/ DDBJ/EMBL on the basis of the strain-derivative alignment. For computation of the molecular pedigree, we used the neighbor-joining method, in which 1,000 bootstraps were generated to test the validity of the topology. For analysis, we used DNASIS Pro (Hitachi Software Engineering, Yokohama). The analysis results of the molecular pedigree are shown in FIG. 5.

We extracted and purified DNAs from cultured bacteria, and obtained DNAs for measuring the G+C content. We used High Performance Liquid Chromatography (HPLC). We added 20 liters of nuclease P1 solution (0.1 mg/mL of nuclease P1 (DNA-GC Kit, made by Yamasa Corporation, sold by Seikagaku Corporation), 40 mM of sodium acetate and 2 mM of zinc sulfate [pH 5.3]) to the same amount of DNAs (350 g/ml) that are dissolved in sterilized water and thermally denatured, and treated the solution for one hour at 50˚C to dissolve it into nucleotides and used it as a sample for HPLC measurement. We performed measurement using an HPLC device (LC-10, Shimadzu, Kyoto) under the following conditions.

Moving phase: 10 mM phosphate buffer (10 mM potassium phosphate, 10 mM monobasic potassium phosphate [pH 7.0]).
Column: Develosil RPAQUEOUS, ϕ4.6 mm x 250 mm (Nomura Chemical, Co., Ltd., Aichi).
Flow speed: 1.5 ml/min.
Detected wavelength: 270 mm.

We identified each nucleotide from the retention times of the standard nucleotide compound (containing equal moles of dCMP, dAMP, dGMP and dTMP) contained in the DNA-GC Kit (made by Yamasa Corporation, sold by Seikagaku Corporation) and the sample. At the same time, we calculated the DNA base composition of the sample expressed as a GC content from the ratio of the peak areas of the standard nucleotide compound and the sample using the following equation.

$$G + C' \text{mol\%} = ((Cx/Cs + Gx/Gs)/(Cx/Cs + Ax/As + Gx/Gs + Tx/Ts)) \times 100$$

Nx: Value of the peak area of dCMP, dAMP, dGMP and dTMP of the sample.
Ns: Value of the peak area of dCMP, dAMP, dGMP and dTMP contained in the standard nucleotide compound.

The results of the G + C content measurement are shown in Table 6.

Table 6
G + C content measurement results

| Sample | GC content (%) |
|---|---|
| 1719-6b | 40.5 |

According to the analysis of the 16S rDNA base sequence, the SIID1719-6b's similarity to its closest relative strain Lactobacillus mali is 95.3%. While the G + C content of Lactobacillus mali is 32.5-33.0%, that of SIID1719-6b is 40.5%. In taxonomy, there is a guideline for two species to be considered the same species. According to this guideline, in order for any two species to be considered the same species, the result of their DNA-DNA hybrid formation test must be 70% or higher. That is the definition of "being the same species". In order for the result of a DNA-DNA hybrid formation test to be 70% or higher, the result of a 16S rDNA base sequence analysis must exceed 97% and the difference in the G + C content between the two species compared must be smaller than 3%. Our test results do not meet these criteria. Accordingly, it has been found that SIID1719-6b is a new species.

## Claims

1. A new species of microorganism having antioxidant properties that is separated from Kefir grains and belongs to Lactobacillaceae SIID 1719-6b having the deposit accession number FERM ABP-10299.

2. A microorganism culture having antioxidant properties obtained in a single culture of the new species of microorganism according to claim 1.

3. A microorganism culture according to claim 2, wherein the bacteria are removed by a centrifugal separator and sterilized using a sterilizing filter.

4. A drink having antioxidant effects added with cultures or bacteria obtained by culturing microorganisms separated from Kefir grains and a new species of microorganism according to claim 1.

5. A food having antioxidant effects added with cultures or bacteria obtained by culturing microorganisms separated from Kefir grains and a new species of microorganism according to claim 1.

6. A cosmetic having antioxidant effects added with cultures or bacteria obtained by culturing microorganisms separated from Kefir grains and a new species of microorganism according to claim 1.

7. An animal feed having antioxidant effects added with cultures or bacteria obtained by culturing microorganisms separated from Kefir grains and a new species of microorganism according to claim 1.

## Patentansprüche

1. Eine neue Mikroorganismenart mit antioxidativen Eigenschaften, die aus Kefirkörnern abgetrennt wird und zu Lactobacillaceae der SITD 1719-6b mit der Hinterlegungseingangsnummer FERM ABP-10299 gehört.

2. Mikroorganismenkultur mit antioxidativen Eigenschaften, die in einer Einzelkultur der neuen Mikroorganismenart gemäß Anspruch 1 erhalten wurde.

3. Mikroorganismenkultur gemäß Anspruch 2, wobei die Bakterien durch eine Trennzentrifuge entfernt werden und durch Verwendung eines Sterilisierfilters sterilisiert wird.

4. Ein Getränk mit antioxidativen Wirkungen, versetzt mit Kulturen oder Bakterien, die durch Kultur von Mikroorganismen, die aus Kefirkörnern abgetrennt wurden, und einer neuen Mikroorganismenart gemäß Anspruch 1 erhalten wurden.

5. Ein Lebensmittel mit antioxidativen Wirkungen, das mit Kulturen oder Bakterien versetzt ist, die durch die Kultur von Mikroorganismen, die aus Kefirkörnern abgetrennt wurden, und einer neuen Mikroorganismenart gemäß Anspruch 1 erhalten wurden.

6. Ein Kosmetikum mit antioxidativen Wirkungen, das mit Kulturen oder Bakterien versetzt ist, die durch die Kultur von Mikroorganismen, die aus Kefirkörnern abgetrennt wurden, und einer neuen Mikroorganismenart gemäß Anspruch 1 erhalten wurden.

7. Ein Tierfutter mit antioxidativen Wirkungen, das mit Kulturen oder Bakterien versetzt ist, die durch die Kultur von Mikroorganismen, die von Kefirkörnern abgetrennt wurden, und einer neuen Mikroorganismenart gemäß Anspruch 1 erhalten wurden.

## Revendications

1. Nouvelle espèce de microorganismes ayant des propriétés antioxydantes qui est extraite de grains de Kéfir et qui appartient aux Lactobacillaceae SIID 1719-6b ayant le numéro de dépôt FERM ABP-10299.

2. Culture de microorganismes ayant des propriétés antioxydantes obtenues dans une seule culture de la nouvelle espèce de microorganismes selon la revendication 1.

3. Culture de microorganismes selon la revendication 2, dans laquelle les bactéries sont éliminées par un séparateur centrifuge et stérilisées au moyen d'un filtre de stérilisation.

4. Boisson ayant des effets antioxydants enrichie de cultures ou bactéries obtenues par la mise en culture de microorganismes extraits de grains de Kéfir et une nouvelle espèce de microorganismes selon la revendication 1.

5. Nourriture ayant des effets antioxydants enrichie de cultures ou bactéries obtenues par la mise en culture de microorganismes extraits de grains de Kéfir et une nouvelle espèce de microorganismes selon la revendication 1.

6. Cosmétique ayant des effets antioxydants enrichi de cultures ou bactéries obtenues par la mise en culture de microorganismes extraits de grains de Kéfir et une nouvelle espèce de microorganismes selon la revendication 1.

7. Nourriture pour animaux ayant des effets antioxydants enrichie de cultures ou bactéries obtenues par la mise en culture de microorganismes extraits de grains de Kéfir et une nouvelle espèce de microorganismes selon la revendication 1.

FIG. 1

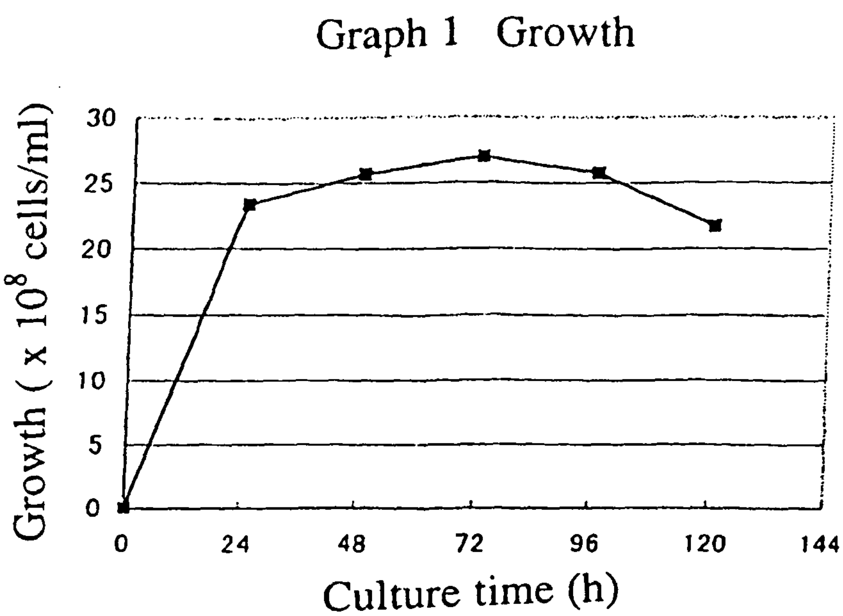
Graph 1 Growth
Growth ( x 10^8 cells/ml)
30
25
20
15
10
5
0
0
24
48
72
96
120
144
Culture time (h)

FIG. 2

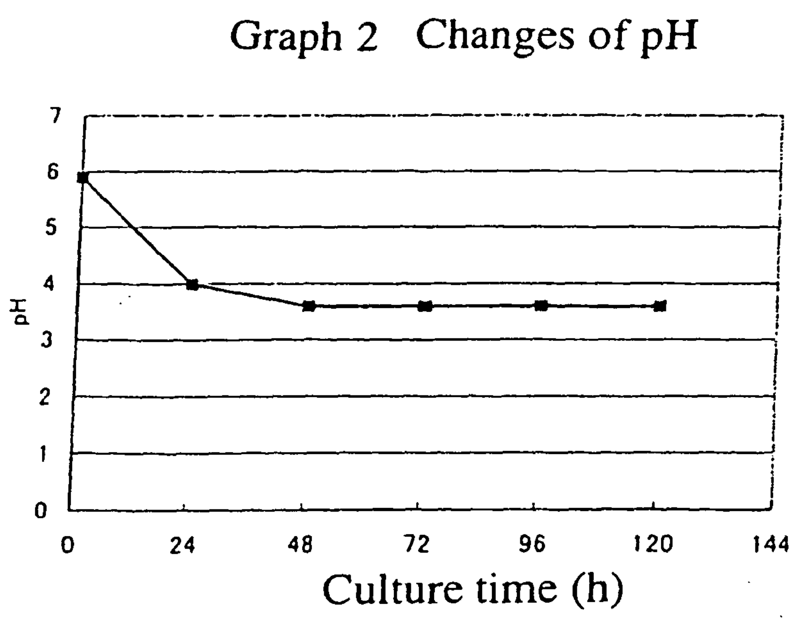
Graph 2 Changes of pH
pH
7
6
5
4
3
2
1
0
0
24
48
72
96
120
144
Culture time (h)

FIG. 3.

Graph 3 Antioxidant properties

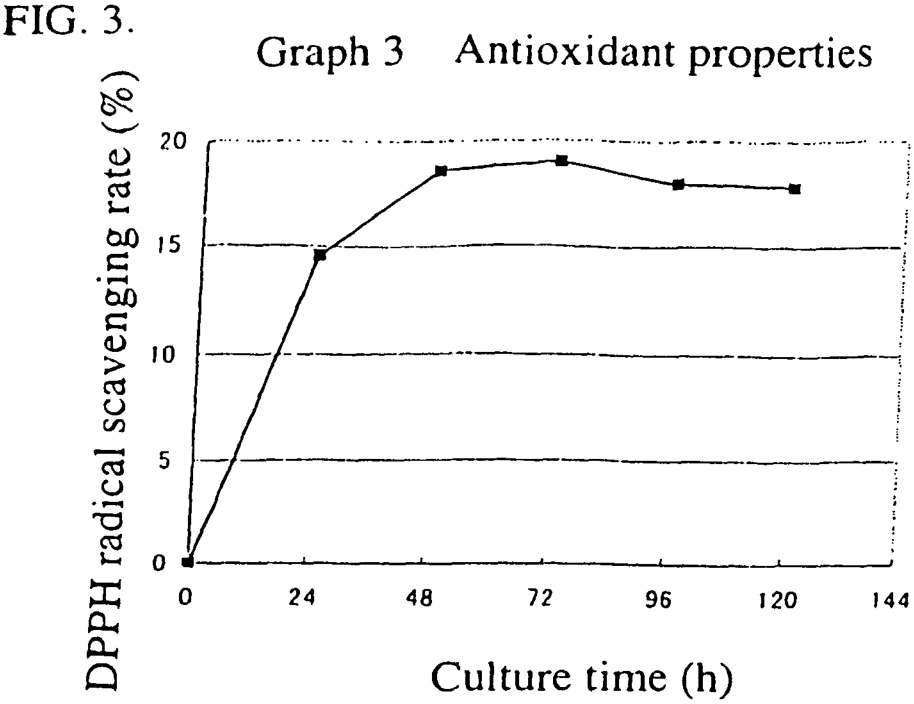

FIG. 4

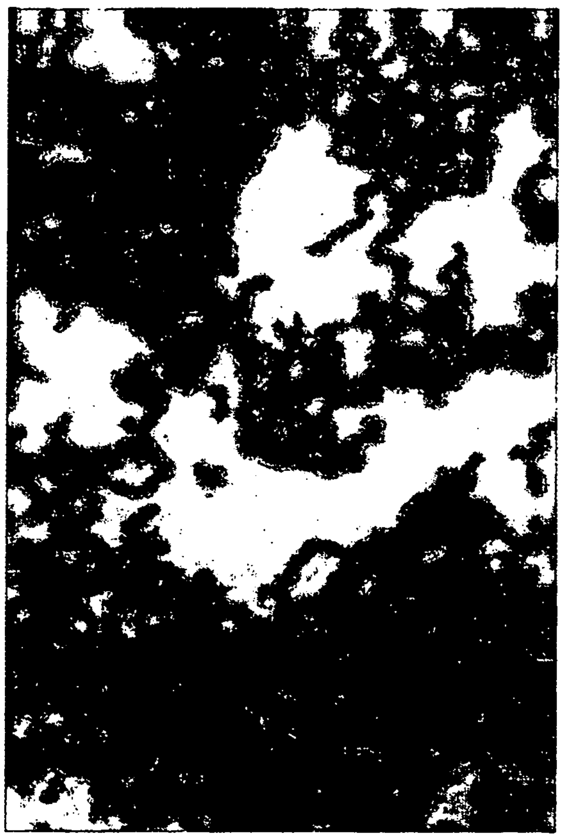

FIG. 5

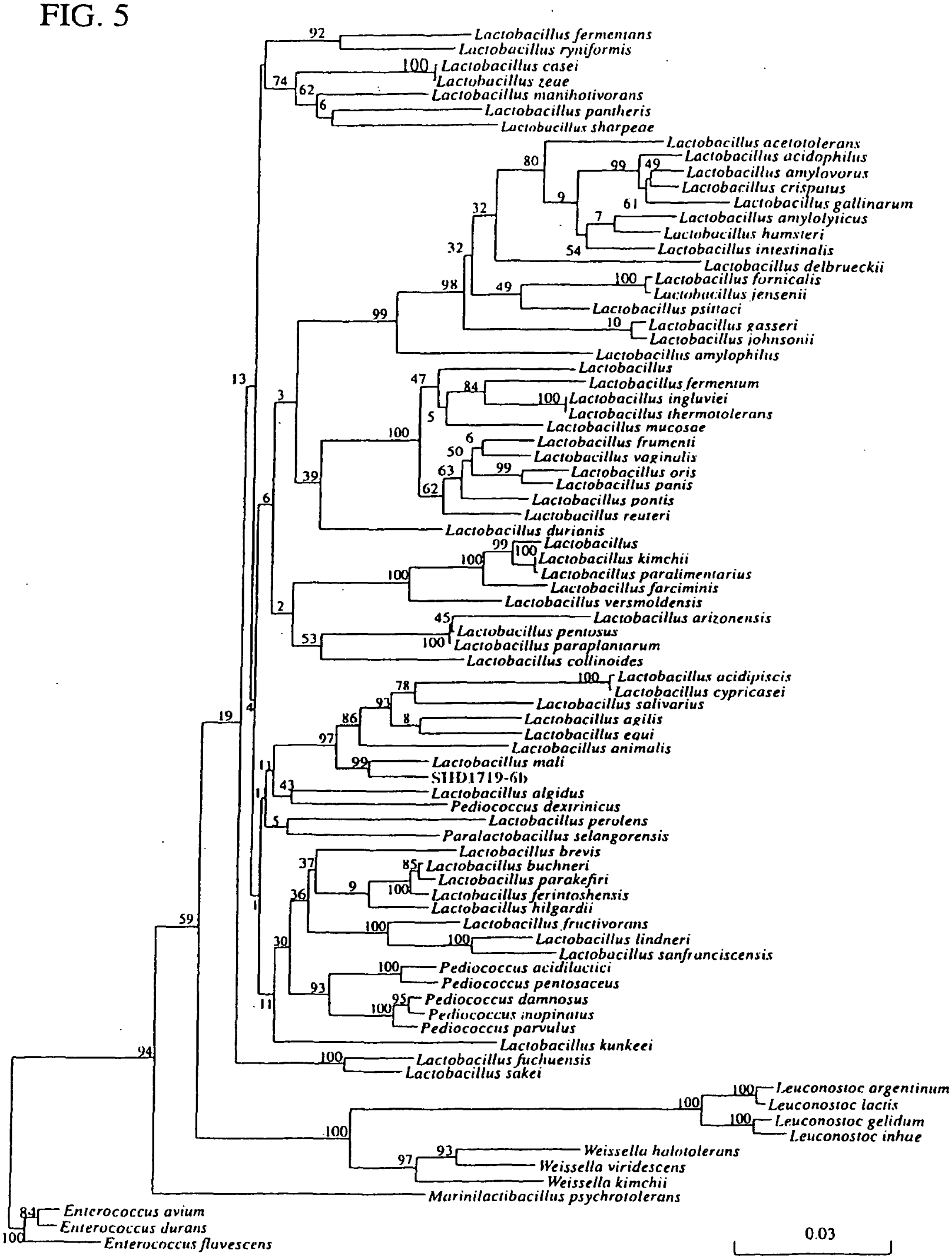
Lactobacillus fermentans
Lactobacillus ryniformis
Lactobacillus casei
Lactobacillus zeae
Lactobacillus manihotivorans
Lactobacillus pantheris
Lactobacillus sharpeae
Lactobacillus acetotolerans
Lactobacillus acidophilus
Lactobacillus amylovorus
Lactobacillus crispatus
Lactobacillus gallinarum
Lactobacillus amylolyticus
Lactobacillus hamsteri
Lactobacillus intestinalis
Lactobacillus delbrueckii
Lactobacillus fornicalis
Lactobacillus jensenii
Lactobacillus psittaci
Lactobacillus gasseri
Lactobacillus johnsonii
Lactobacillus amylophilus
Lactobacillus
Lactobacillus fermentum
Lactobacillus ingluviei
Lactobacillus thermotolerans
Lactobacillus mucosae
Lactobacillus frumenti
Lactobacillus vaginalis
Lactobacillus oris
Lactobacillus panis
Lactobacillus pontis
Lactobacillus reuteri
Lactobacillus durianis
Lactobacillus
Lactobacillus kimchii
Lactobacillus paralimentarius
Lactobacillus farciminis
Lactobacillus versmoldensis
Lactobacillus arizonensis
Lactobacillus pentosus
Lactobacillus paraplantarum
Lactobacillus collinoides
Lactobacillus acidipiscis
Lactobacillus cypricasei
Lactobacillus salivarius
Lactobacillus agilis
Lactobacillus equi
Lactobacillus animalis
Lactobacillus mali
SHD1719-6b
Lactobacillus algidus
Pediococcus dextrinicus
Lactobacillus perolens
Paralactobacillus selangorensis
Lactobacillus brevis
Lactobacillus buchneri
Lactobacillus parakefiri
Lactobacillus ferintoshensis
Lactobacillus hilgardii
Lactobacillus fructivorans
Lactobacillus lindneri
Lactobacillus sanfranciscensis
Pediococcus acidilactici
Pediococcus pentosaceus
Pediococcus damnosus
Pediococcus inopinatus
Pediococcus parvulus
Lactobacillus kunkeei
Lactobacillus fuchuensis
Lactobacillus sakei
Leuconostoc argentinum
Leuconostoc lactis
Leuconostoc gelidum
Leuconostoc inhae
Weissella halotolerans
Weissella viridescens
Weissella kimchii
Marinilactibacillus psychrotolerans
Enterococcus avium
Enterococcus durans
Enterococcus flavescens
0.03

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002315520 A
- JP 2004149442 B
- JP 2004175369 A